## Europäisches Patentamt
## European Patent Office
## Office européen des brevets

(11) Publication number: **0 131 456**
**A2**

(12) # EUROPEAN PATENT APPLICATION

(21) Application number: **84304653.3**

(22) Date of filing: **06.07.84**

(51) Int. Cl.⁴: **C 12 N 15/00**
C 12 P 19/42, C 12 N 1/20
A 61 K 31/68

(30) Priority: **08.07.83 JP 124273/83**
**08.07.83 JP 124274/83**

(43) Date of publication of application:
**16.01.85 Bulletin 85/3**

(84) Designated Contracting States:
**CH DE FR GB LI**

(71) Applicant: **KUREHA KAGAKU KOGYO KABUSHIKI KAISHA**
**9-11 Horidome-cho 1-chome Nihonbashi Chuo-ku Tokyo(JP)**

(72) Inventor: **Takeuchi, Daizou**
**5-11-3 Nakaoka-machi Iwaki-shi Fukushima-ken(JP)**

(72) Inventor: **Hamada, Masahiro**
**55-20 Tsurumaki Takakura-machi Iwaki-shi Fukushima-ken(JP)**

(74) Representative: **Myerscough, Philip Boyd et al,**
**J.A.Kemp & Co. 14, South Square Gray's Inn London, WC1R 5EU(GB)**

(54) **Production of vitamin B12 employing a fused cell hybrid.**

(57) A fused cell hybrid is obtained by fusing a cell of a chlorophyll-producing bacterial species belonging to the photosynthetic genus *Rhodopseudomonas*, such as *Rhodopseudomonas spheroides*, with a cell of a vitamin $B_{12}$- producing bacterial species belonging to the methanol-metabolising genus *Protaminobacter*, such as *Protaminobacter ruber*. The fused cell hybrid is cultured to produce vitamin $B_{12}$.

Figure

- 1 -

TITLE: PRODUCTION OF VITAMIN $B_{12}$ EMPLOYING A FUSED CELL HYBRID

The present invention relates to a fused cell hybrid having a high productivity of vitamin $B_{12}$ (cobalamin), to a process for producing the hybrid and further to a process for preparing vitamin $B_{12}$ by culturing the hybrid.

More in detail, the present invention relates to a fused cell hybrid obtained by fusing a cell of a photosynthetic microorganism of Rhodopseudomonas spheroides with a cell of a methanol-assimilating microorganism of Protaminobacter ruber.

More in detail, the present invention relates to (1) Rhodopseudomonas protamicus itself, (2) a process for originating Rhodopseudomonas protamicus, comprising subjecting a photosynthetic bacterial species, Rhodopseudomonas spheroides and a methanol-assimilating bacterial species, Protaminobacter ruber, to bacterial cell fusion and (3) a process for fermentatively producing vitamin $B_{12}$, comprising culturing a strain of the originated Rhodopseudomonas protamicus and collecting vitamin $B_{12}$ produced by the strain and accumulated within and outside the bacterial bodies thereof.

It has been well known that vitamin $B_{12}$ participates in metabolism of various substances such as nucleic acids, proteins, amino acids such as methionine and lipids within living bodies. In recent years, utilization of vitamin $B_{12}$ as a medicine has been increased for treating the disturbance of myelopoietic function, the hepatic disturbance and the disease of nerve system other than the treatment and prevention of the deficiency diseases of vitamin $B_{12}$. Furthermore, various

physiological functions of vitamin $B_{12}$ such as the remarkable immunity-regulating function of methyl-vitamin $B_{12}$ have been elucidated so that an importance of vitamin $B_{12}$ is increased.

If vitamin $B_{12}$ is produced by an organic synthesis, the yield is low and the cost of the produced vitamin $B_{12}$ is very expensive because the chemical structure of vitamin $B_{12}$ is very complicated and a stereoisomeric structure exists in the side chain of corrin-ring in vitamin $B_{12}$ so that the large number of synthetic steps are required. Accordingly, vitamin $B_{12}$ is exclusively produced by a fermentative process.

However, even when vitamin $B_{12}$ is produced by the fermentative process, it is expensive because of the low productivity of the hitherto known bacteria producing vitamin $B_{12}$. The utilization of bacteria belonging to Propionibacterium, Bacillus, Corynebacterium, Arthrobacter, Achromobacter, Klebsiella, Rhodopseudomonas, Pseudomonas, Protaminobacter, Streptomyces, Rhodospirillum, Actinomyces, Cellulomonas, Ruminantium, Nocardia or Methanomonas has been known for producing vitamin $B_{12}$. The productivity of vitamin $B_{12}$ in the known bacteria is at most about 0.2 to about 23 mg/ℓ of the liquid culture medium and is not satisfiable in industrial production. Accordingly, it is demanded to research for a microorganism having a high productivity of vitamin $B_{12}$.

As a result of the present inventors' studies for producing a microorganism having a high productivity of vitamin $B_{12}$ by cell fusion, the present inventors have attained the

- 3 -

present invention.

It is an object of the present invention to provide a fused cell hybrid having a productivity of vitamin $B_{12}$, obtained by fusing a cell of a microorganism of Rhodopseudomonas spheroides with a cell of a microorganism of Protaminobacter ruber. Another object of the present invention is to provide a process for producing a fused cell hybrid having a productivity of vitamin $B_{12}$, which comprises fusing a cell of a microorganism of Rhodopseudomonas spheroides with a cell of a microorganism of Protaminobacter ruber. A further object of the present invention is to provide a process for preparing vitamine $B_{12}$, which comprises

culturing a fused cell hybrid having a productivity of vitamin $B_{12}$, said hybrid being obtained by fusing a cell of a microorganism of Rhodopseudomonas spheroides with a cell of a microorganism of Protaminobacter ruber, and

collecting vitamin $B_{12}$.

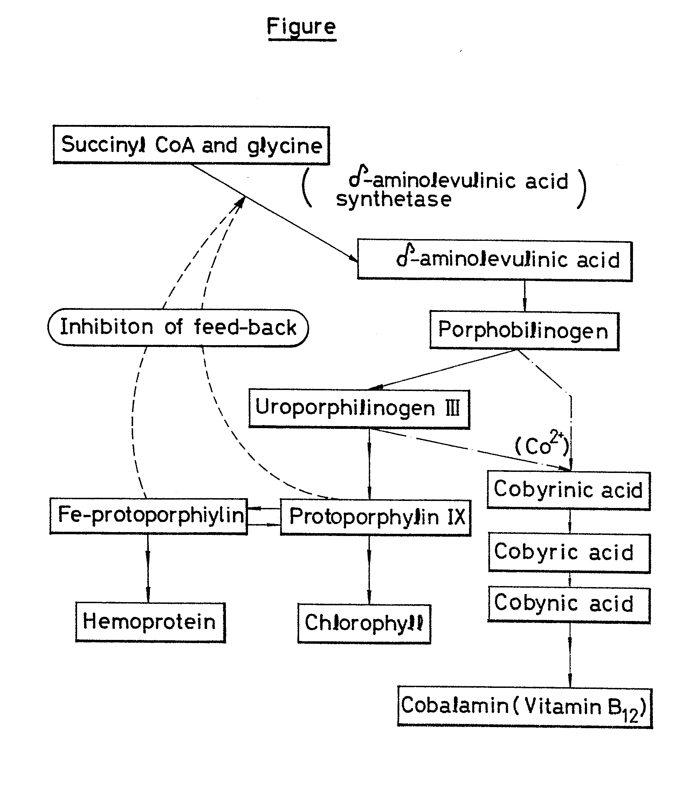

In the drawing, the single figure is a flowchart of biosynthesis of vitamin $B_{12}$.

The hybrids of the present invention have a productivity of vitamin $B_{12}$ of about 135 mg/ℓ of liquid culture medium or higher than about 135 mg/ℓ of liquid culture medium.

In the process for producing a fused cell hybrid of the present invention, protoplasts of the cells may be formed by the known method, for example, the method described in Agric. Biol. Chem., **45** (6), 1515 (1981). However, it is preferable to utilize a modified method of the known method, wherein the amount of lysozyme is larger than that of the known method and the freezing treatment is not carried out in the present invention.

As the bacterial cells for use in fusing treatment of the formed protoplasts, the cells in any stage may be used, if the cells are the complete bacterial bodies. Nevertheless, those bacterial bodies in the latter period of the logarithmic growth phase are preferable, since the efficiency of the production of protoplasts is increased.

For culturing the bacterial bodies, a general culture medium is used in accordance with the conventional procedures for culturing a bacteria. The treatment of cell fusion in the present invention is carried out in accordance with the publicly known method except for adding methanol to the culture medium.

In general, the artificial origination of a bacterial strain by cell fusion has been regarded to be extremely difficult because the stabilization of the characters given to the originated bacterial·cells is very difficult. There is a rare occurrence of origination in strains obtained by cell fusion, in which the character(s) given by the cell fusion has been stabilized without specified treatment. In many cases, various treatments have been tried to stabilize the character(s) given to the strain originated by the cell fusion.

In order to stabilize such character(s) given to the newly originated bacterial strain by cell fusion according to the present invention, it is preferable to expose the cells to ultraviolet rays, for instance, for 20 to 300 sec, more preferably for 30 to 250 sec to the ultraviolet rays from two 10-W ultraviolet lamps at a distance of 30 cm from the cells and as a result, the preferable bacterial strain is obtained in which the character(s) given by the cell fusion is stabilized.

By the way, it has been already known that one of the parent microorganism, Protaminobacter ruber, has a productivity of vitamin $B_{12}$, however, it is only 0.2 to 1.0 mg/$\ell$ of liquid culture medium.

As will be seen in the attached drawing, vitamin $B_{12}$ is biosynthetically produced from glycine and succinyl-CoA via δ-aminolevulinic acid, porphobilinogen and cobyrinic acid, or via δ-aminolevulinic acid, porphobilinogen, uroporphyrinogen III and cobyrinic acid.

In the biosynthetic system, protoporphyrin IX is produced in a small amount from uroporphyrinogen III and then, is accumulated in the system. The accumulation of protoporphyrin IX inhibits δ-aminolevulinic acid synthetase and therefore, the preparation of δ-aminolevulinic acid is suppressed. It is considered that this mechanism has a relationship to the low productivity of vitamin $B_{12}$ in the biosynthetic system.

Another one of the parent microorganism, Rhodopseudomonas spheroides, is a photosynthetic bacteria and is high in the productivity of chlorophyll. Chlorophyll is also produced from glycine and succinyl-CoA biosynthetically via δ-aminolevulinic acid, porphobilinogen, uroporphyrinogen III and protoporphyrin

IX, as vitamin $B_{12}$. Since <u>Rhodopseudomonas</u> <u>spheroides</u> produces a large amount of chlorophyll in spite of the production of protoporphyrin IX, it is considered that the δ-aminolevulinic acid synthetase is not inhibited by the production of protoporphyrin IX.

A fused cell line according to the present invention is obtained by fusing a cell of the microorganism of <u>Rhodopseudo-</u> <u>monas</u> <u>spheroides</u> with a cell of the microorganism of <u>Protaminobacter</u> <u>ruber</u>, and selecting a fused cell hybrid having a high productivity of vitamin $B_{12}$. The selected hybrid of the present invention retains a productive character of vitamin $B_{12}$ according to <u>Protaminobacter</u> <u>ruber</u> and is provided with a character that δ-aminolevulinic acid synthetase is not inhibited by protoporphyrin IX.

One of the fused cell hybrid of the present invention has been deposited on September 10, 1982 in Fermentation Research Institute Agency of Industrial Science and Technology under Budapest Treaty on the international recognition of the deposit of microorganisms for the purposes of patent procedure. The accession number given by the International Depository Authority of the hybrid of the present invention is FERM BP-180.

The fused cell line of the present invention is novel and is named as "<u>Rhodopseudomonas</u> <u>protamicus</u>".

As the parent bacterial species for use according to the present invention, the above-mentioned pair of bacterial species may be mentioned as a preferable example. In addition, every chlorophyll-producing bacterial species belonging to the photosynthetic genus, _Rhodopseudomonas_, as one of the pair may be used and every cobalamin-producing bacterial species belonging to the methanol-metabolizing genus, _Protaminobacter_, as the other of the pair may be used.

The results of observation of the morphological and physiological properties of _Rhodopseudomonas protamicus_ according to the present invention are as follows:

(a) Morphological appearance (after being cultured for 4 days at 30°C in a bouillon-agar culture medium):

    1.  Shape and size:  bacillus of 0.5 to 1.25 micrometer in width and 1.0 to 6.0 micrometer in length.

    2.  Polymorphism:  none

    3.  Motility:  none (also in H-II culture medium and bouillon liquid culture medium)

    4.  Spore-formation: none

    5.  Gram-staining:  negative

    6.  Acid-resistance: none

(b) Growth state in one of the following culture mediums:

  1.  Plate bouillon-agar culture medium (after being cultured for 4 days at 30°C):

    Growth state:  viscous, circular entire colonies with a moderate height

    Colour:  red in the center of the colony and translucent pink in the periphery of the colony

Luster:                yes

Diffusible pigment:  none

2. Slant bouillon-agar culture medium (after being cultured for 4 days at 30°C):

Growth state:        viscous thread-like colonies

Colour:              pale red

Luster:              yes

Diffusible pigment:  none

3. Synthetic agar culture medium (H-II agar culture medium, after being cultured for 14 days at 30°C):

Size of flat colonies: at most 9 mm in diameter

Shape of protuberance on the surface of the colony:

                     circular convex

Colour:              reddish purple

Luster:              yes

Diffusible pigment:  none

4. Liquid bouillon culture medium (after being cultured for 7 days at 30°C under a fluorescent light):

Growth on the surface of the medium: none

Turbidity:           only a slight

Precipitated matter: only a small amount

Colour:              red

5. Stab culture in solid bouillon-gelatine culture medium (after being cultured for 7 days at 30°C):

Growth state:        poor

Liquefaction of gelatin: none

6. Litmus milk (after being cultured for 7 days at 30°C):

   Litmus reaction:      neutral

   Generation of gas:    none

   Solidification or liquefaction: none

(c) Physiological properties:

1. Reduction  of  a nitrate salt: yes

2. Denitrification:       none

3. MR-test:               negative

4. VP-test:               negative

5. Formation of indole:   none

6. Formation of hydrogen sulfide: yes

7. Hydrolysis of starch:  yes, however, weak

8. Utilization of citric acid: none

9. Utilization of inorganic nitrogen source:

   ammonium sulfate:     ±

   urea:                 ±

   ammonium nitrate:     +

   ammonium carbonate:   +

   ammonium chloride:    ±

   ammonium phosphate:   +

10. Utilization of organic nitrogen source:

   yeast extract:        +

   meat extract:         +

   malt extract:         +

   polypeptone:          +

   casamino acid:        +

   corn steep liquor:    +

- 10 -

11. Formation of pigment:

   It forms a water-soluble carotenoid pigment and a
   water-soluble porphyrin series pigment.

12. Presence of urease:     -

13. Presence of oxidase:    +

14. Presence of catalase:   +

15. Environmental conditions for growth:

   pH:                   in a range of from 6.0 to 8.8

   optimum pH:           in a range of from 7.0 to 7.5

   temperature:          in a range of from 20 to 44°C

   optimum temperature:  in a range of from 30 to 37°C

16. Behavior to oxygen:

   It grows in the presence of oxygen in the light and
   in the dark and also it grows in the absence of oxygen
   in the light.

17. Result of O-F test:     fermentative

18. Formation of an acid and a gas from the following sugars:

| Sugar | Acid | Gas |
|---|---|---|
| L-arabinose | - | - |
| D-xylose | ± | - |
| D-glucose | - | - |
| D-mannose | - | - |
| D-fructose | ± | - |
| D-galactose | - | - |
| maltose | - | - |
| sucrose | - | - |

- 11 -

lactose        -        -

trehalose        -        -

D-sorbitol        -        -

D-mannitol        -        -

inositol        -        -

glycerol        -        -

starch        -        -

(d) Utilization of the other carbon source:

methanol:        yes, however, slightly

(e) Susceptibility to antibiotics (by Paper disk method):

| Microorganism | | Antibiotic | | |
|---|---|---|---|---|
| | | Colistin sulfate 150 U (titer) | Bacitracin 2 U (titer) | Cephalothin 30 µg |
| Hybrid | Rhodopseudomonas protamicus FERM BP-180 | r | r | s |
| Parents | Rhodopseudomonas spheroides IFO 12203 | s | s | r |
| | Protaminobacter ruber IFO 3708 | r | r | s |

Note: r;means resistant (non-susceptible) and s;means susceptible.

As the culture medium for culturing microorganism of _Rhodopseudomonas_ _protamicus_ originated by the present invention, any synthetic- or artificial culture medium may be used provided that the culture medium contains the sources for carbon and nitrogen, inorganic salts and minute amounts of nutrients necessary for the growth of the microorganism in the respective proper amounts. As the carbon source, one of various carbohydrates such as glucose, fructose, sucrose, maltose, mannose, starch, aqueous solution of hydrolysed starch and molasses; sugar alcohols such as glycerol; various organic carboxylic acids such as pyruvic acid, fumaric acid, malic acid, succinic acid, lactic acid, acetic acid and the like; or methanol may be used. As the nitrogen source, ammonia, one of various inorganic ammonium salts and organic ammonium salts such as ammonium phosphate, ammonium chloride, ammonium sulfate, ammonium carbonate, ammonium nitrate and ammonium acetate, one of nitrogen-containing substances such as urea; one of nitrogen-containing organic substances such as peptone, NZ-amine, malt extract, meat extract, yeast extract, corn steep liquor, casein hydrolyzate, fish meal and digested fish meal, amino acids, de-fatted soy bean cakes and digested products thereof may be used. It is preferable to use the culture medium containing a large amount of the nitrogen source which contains a large amount of available nitrogen to the microorganism so as to obtain a large amount of vitamin $B_{12}$. As the inorganic salts, dipotassium monohydrogen phosphate, potassium dihydrogen phosphate, magnesium sulfate,

- 13 -

sodium chloride, ferrous sulfate, calcium chloride, zinc sulfate, manganese sulfate and cobalt chloride may be used.

The presence of divalent cobalt ions in the culture medium is inevitably required and the concentration of divalent cobalt ions in the culture medium is related to the yield of vitamin $B_{12}$ produced by the hybrid. The yield of vitamin $B_{12}$ is extremely high in the culture medium in the case where an amount of divalent cobalt ions corresponding to 10 to 100 mg of $CoCl_2 \cdot 6H_2O$ are present in one litre of liquid culture medium. In addition, the presence of the other metallic ions affects the production of vitamin $B_{12}$. The addition of both divalent calcium ions and ferrous ions or the addition of three divalent ions of divalent calcium ions, ferrous ions and divalent manganese ions acts in accelerating the production of vitamin $B_{12}$.

Furthermore, the production of vitamin $B_{12}$ is accelerated by the addition of a vitamin such as vitamin $B_1$, biotin and nicotinic acid and by the addition of an amino acid such as methionine, glycine and serine to the culture medium.

In the case where the microorganism requires the other nutritive substances such as the other vitamins, amino acids, inorganic salts, organic salts and minerals, it is necessary to add the required nutritive substances to the culture medium, however, in the case where the components of the culture medium contain such indispensable substances, it is not necessary to add such nutritive substances.

As a concrete example of the culture mediums for culturing a microorganism of Rhodopseudomonas protamicus of the

- 14 -

present invention in order to produce vitamin $B_{12}$, the following culture medium may be mentioned. This culture medium is hereinafter referred to as H-II culture medium:

| | |
|---|---|
| glucose | 10 g |
| polypepton | 10 g |
| yeast extract | 10 g |
| urea | 1.5 g |
| ammonium sulfate | 1.5 g |
| potassium dihydrogen phosphate | 0.5 g |
| dipotassium monohydrogen phosphate | 2.5 g |
| magnesium sulfate heptahydrate | 0.3 g |
| calcium chloride dihydrate | 10 mg |
| zinc sulfate heptahydrate | 10 mg |
| ferrous sulfate heptahydrate | 10 mg |
| manganese sulfate heptahydrate | 1 mg |
| cobalt chloride hexahydrate | 100 mg |

The components of the culture medium are dissolved in de-ionized water to obtain one litre of the culture medium having pH 7.0.

- 15 -

Of course, the obtained culture medium is one of concrete examples for use in the present invention. The other suitable nutrients, minute ingredients and precursor of vitamin $B_{12}$, for instance, 5,6-dimethylbenzimidazole as a constituent of the lower ligand in vitamin $B_{12}$, may be added to the culture medium.

Rhodopseudomonas protamicus of the present invention may be cultured by stationary culture, shaking culture, anaerobic culture under illumination or spinner culture under aeration. The culture is carried out generally at a culturing temperature of 20 to 44°C, preferably 30 to 37°C and at a pH of the culture medium of 6.0 to 8.8, preferably at a controlled pH in a range from 7.0 to 7.5 by the addition of a pH-buffer and/or a neutralizing agent for 2 to 7 days. In ordinary cases of such a culture, both the growth rate of the bacterial bodies and the production rate of vitamin $B_{12}$ attain their maximum by 3 to 5 days.

Although a part of the produced vitamin $B_{12}$ by the microorganism is contained in the culture medium used therefor, a larger part thereof is contained in the proliferated bacterial bodies. Accordingly, in order to detect vitamin $B_{12}$, the pH of the whole cultured matter is adjusted to 6.0 by the addition of sulfuric acid thereto and potassium cyanide is added to the adjusted whole cultured matter so as to bring the concentration of potassium cyanide in the resultant mixture finally to 0.01 % by weight, and the prepared mixture is treated in autoclave for 15 min under a pressure of 1.2 $kg/cm^2$. Thereafter, the treated mixture is subjected to a conventional microbiological method for quantitatively determining vitamin $B_{12}$ by the use of Lactobacillus leichmannii to detect and determine the amount of vitamin $B_{12}$ in the whole mixture.

In order to detect and determine vitamin $B_{12}$ contained in the proliferated bacterial bodies, the whole cultured matter is subjected to centrifugal separation, the separated bacterial bodies is washed with water, the washed bacterial bodies are subjected to treatment in autoclave in the same manner as above and then the treated matter is subjected to the same procedures of detecting and determining vitamin $B_{12}$ as above. Besides, after washing the bacterial bodies, 100 times by weight of 80% aqueous ethanolic solution (that is, a mixture of 80% by weight of ethanol and 20% by weight of water) containing 0.01 % of potassium cyanide are added to the washed bacterial bodies and the pH of the mixture is adjusted to 6.0, and the adjusted mixture is extracted by aqueous ethanol while stirring the mixture for 20 min at 88 to 90°C.

The whole system is cooled to room temperature after extraction and the extraction residue is removed by centrifugal separation. Ethanol is distilled off from the obtained supernatant at 40°C under a reduced pressure and the obtained residual matter is subjected to the above-mentioned conventional microbiological method for quantitatively determining vitamin $B_{12}$ by using Lactobacillus leichmannii.

The collection of vitamin $B_{12}$ from the whole cultured matter may be carried out by the following conventional process for isolating and purifying vitamin $B_{12}$. For instance, the whole cultured matter is at first subjected to centrifugal treatment for separating into the bacterial bodies and the supernatant liquid, and vitamin $B_{12}$ is obtained from each of the separated fractions by the following steps:

(1) Steps for obtaining vitamin $B_{12}$ from the bacterial bodies:

In order to separate vitamin $B_{12}$ from the bacterial bodies without changing the state thereof in the bacterial body, i.e., methylcobalamin and/or 5,6-dimethylbenzimidazole-cobamide coenzyme (hereinafter referred to as DBCC) as a solid or syrup-like material, the bacterial bodies are boiled in water regulated to pH of 5.0 by sulfuric acid to extract vitamin $B_{12}$. After cooling the treated mixture, the bacterial bodies are removed by centrifugation and the supernatant is condensed to obtain the object product. Besides, the bacterial bodies are subjected to extraction with 80% aqueous ethanolic solution by boiling for 20 min at 100°C to obtain vitamin $B_{12}$. Further, vitamin $B_{12}$ may be extracted from the bacterial bodies with 20 % aqueous solution of pyridine (that is, a mixture of 20 % by weight of pyridine and 80 % by weight of water).

On the other hand, in order to separate vitamin $B_{12}$ as a stable compound, that is cyanocobalamin, the bacterial bodies are extracted with an aqueous solution containing cyanide ions or an aqueous ethanolic solution containing cyanide ions

- 18 -

under heating by the conventional method and the liquid extract is condensed to obtain vitamin $B_{12}$ as cyanocobalamin.

However, in order to obtain a highly purified product of vitamin $B_{12}$, it is preferable to subject the liquid extract to the treatment such as extraction with phenol, the treatment with activated carbon or ion-exchanger such as ion-exchange resin, ion-exchange cellulose and fractionation according to molecular weight or to the combination thereof. An aqueous solution containing vitamin $B_{12}$ at a relatively high concentration is obtained by the above-mentioned treatment and then vitamin $B_{12}$ is obtained as pure crystals by adding an organic solvent such as acetone to the aqueous solution.

(2) Steps for obtaining vitamin $B_{12}$ from the supernatant:

The supernatant liquid obtained from the cultured matter by centrifugal separation is, at first, subjected to condensation to dryness and thereafter, the obtained condensate is subjected to the series of extraction and purification in the same manner as those used in the extraction of vitamin $B_{12}$ from the bacterial bodies so as to obtain purified vitamin $B_{12}$.

The present invention relates to a process for fermentatively producing vitamin $B_{12}$ at a high concentration by the newly originated microorganism obtained by cell fusion of two kinds of microorganisms based on the new idea of the present inventors concerning the combination of microbial biosynthetic capabilities of two microorganisms by cell fusion

- 19 -

thereof and the utilization of the two metabolic pathways thereof.

The process of the present invention can produce vitamin $B_{12}$ economically in a large scale and accordingly, the present invention possesses a high industrial merit.

The present invention will be explained more in detail while referring to the following non-limitative examples:

In addition, the amount and the purity of vitamin $B_{12}$ shown in Examples have been determined by the following methods while comparing to those of a commercialized reagent-grade vitamin $B_{12}$ (made by Wako Pure Chemical Co., Ltd.).

Methods for determining the amount and purity of vitamin $B_{12}$:

1.  A publicly known microbiological method for quantitatively determining vitamin $B_{12}$ while using Lactobacillus leichmannii ATCC 7830:

    Refer to "Principles and Basic Procedures in Microbiological Determination of Vitamins and Amino Acids" by Etsuko Kudo as "Nissan Library No. 2", Ed. 1978 by Nissui Seiyaku Co., Ltd.

2.  A spectrocolorimetric method while utilizing absorbance of vitamin $B_{12}$:

    The specimen is diluted with distilled water in a mess-flask exactly to 10 ml, the diluted solution is introduced into a cuvette of a spectrocolorimeter of 1 cm in light path, and the absorbance of the diluted solution is measured at $361 \pm 1$ nm while using pure water as a control, the absorbance being $A_{361}$.

- 20 -

The amount of vitamin $B_{12}$ (cyanocobalamin) in 10 ml of the aqueous solution is obtained by the following formula and represented by "Amount":

$$\text{Amount (microgram)} = A_{361} \times \frac{100}{0.207}$$

wherein the coefficient, 0.207, is an absorbance at $361 \pm 1$ nm of an aqueous solution of 100 micrograms of the authentic cyanocobalamin in 10 ml of pure water in a cuvette of 1 cm of light path.

3.  A liquid-chromatographic method:

| | |
|---|---|
| Apparatus: | Liquid Chromatograph (made by Waters Associates, Model 440) |
| Column: | 4 mm in inner diameter and 300 mm in length, packed with μ-Bondapak $C_{18}$ (made by Waters Associates). |
| Column temperature: | room temperature |
| Wave length used for detection: | 254 nm |
| Moving phase: | a mixture of 0.04M aqueous tartaric acid-disodium hydrogen phosphate buffer solution (pH 3.0) and methanol, the concentration of methanol being 37 % by volume. |

EXAMPLE 1:

Origination of Fused Cell Hybrids by Cell Fusion of Two
Mutually Different Microorganism

1-1: Formation of Protoplast:

In 10 ml of H-II culture medium preliminarily sterilized in an autoclave for 20 min at 120°C, the microorganism of Rhodopseudomonas spheroides (deposited in the Institute for Fermentation Osaka, Japan under the deposition number of IFO 12203) was inoculated and was cultured for 24 hours at 30°C while shaking the inoculated culture medium.

The cultured bacteria as the mother bacteria were inoculated to a new H-II culture medium preliminarily treated as above at a volume concentration of 1 % and were subjected to shaking culture for 15 hours at 30°C.

The cultured bacterial bodies at the later period of logarithmic proliferation stage were collected while avoiding contamination by the other kind of bacteria and were washed with sterilized water. The following procedures were carried out while avoiding contamination by the other kind of bacteria. The washed bacterial bodies were suspended in 10 ml of 0.1 M-tris· hydrochloric acid buffer solution of pH 8.0, which had been preliminarily sterilized in an autoclave for 20 min at 120°C and contained 1 mM of ethylenediaminetetraacetic acid (EDTA) and 20% of sucrose. Then, 10 mg of abacterially filtered lysozyme was added thereto and the added suspension was subjected to shaking treatment for 30 min at 30°C under 180 r.p.m.

On the other hand, the protoplast of Protaminobacter ruber (deposited in the Institute for Fermentation Osaka, Japan

0131456

- 22 -

under the deposition number IFO 3708) was prepared as follows. In 10 ml of H-II culture medium preliminarily sterilized in an autoclave for 20 min at 120°C, the microorganism of _Protaminobacter ruber_ was inoculated and was cultured for 24 hours at 30°C while shaking the inoculated culture medium. The cultured bacteria as the mother bacteria were inoculated to a new H-II culture medium preliminarily treated as above at a volume concentration of 1 % and were subjected to shaking culture for 15 hours at 30°C. The cultured bacterial bodies at the later period of logarithmic proliferation stage were collected and were washed with sterilized water. The washed bacterial bodies were suspended in 10 ml of 0.1 M-tris·hydrochloric acid buffer solution of pH 8.0, which had been preliminarily sterilized in an autoclave for 20 min at 120°C and contained 1 mM of EDTA and 20 % of sucrose. Thereafter, 10 mg of abacterially filtered lysozyme was added to the suspension and further, methanol was added thereto so that the final concentration of methanol is 0.2 % and then, the added suspension was subjected to shaking treatment for 30 min at 30°C under 180 r.p.m.

Just after the treatment, an aqueous suspension containing $10^3$ cells of the formed protoplast of _Rhodopseudomonas spheroides_ IFO 12203 in one millilitre thereof was prepared by adjusting the cell concentration in the treated suspension with the addition of the buffer solution.

In quite the same manner as above an aqueous suspension containing $10^3$ cells of the obtained protoplast of _Protaminobacter_

- 23 -

ruber IFO 3708 in one millilitre was obtained.

1-2:  Treatment for Cell Fusion of Protoplast:

One millilitre of the prepared aqueous suspension of the protoplasts of Rhodopseudomonas spheroides IFO 12203 was mixed with one millilitre of the prepared aqueous suspension of the protoplasts of Protaminobacter ruber IFO 3708.  While maintaining the mixture at 5°C, the mixture was subjected to centrifugal separation for 10 min at 8000 r.p.m., and after removing the supernatant liquid, 3 ml of 40 % aqueous solution of polyethylene glycol having a molecular weight of 4000 (that is, a mixture of 40 % by weight of polyethylene glycol and 60 % by weight of water) were added to the residue.  The prepared aqueous suspension was subjected to cell fusion treatment for one min at 0°C.

1-3:  Regeneration and Culture of the Cell Fused Hybrid Obtained by Cell Fusion:

The aqueous suspension obtained by the treatment for protoplast fusion was diluted with the same buffer solution mentioned in Example 1-1 and the diluted suspension was added to a preliminarily adjusted and sterilized bouillon-sucrose hypertonic buffer solution containing 0.4 % of methanol and an antibiotic.  The prepared mixture was scattered onto a flat plate of 1.5 to 2.0 % of agar and after piling a soft layer of 0.5 % agar which was kept at about 37 to 40°C on the flat plate, the piled layers were cultured for one week at 30°C to form a few bacterial colonies.

The few bacterial colonies appeared on the agar layers.  Each colony was separately inoculated on a H-II agar culture medium. After separating single colony recognized to have originated by the cell fusion, each of the separated colonies was subjected to shaking culture in a H-II liquid culture medium at 30°C for 16 hours.

Then, each cultured matter was diluted with sterilized water and after plating out the diluted matter on a H-II agar culture medium, the treated agar culture medium was exposed to ultraviolet rays from two 10 W luminescent lamps at a distance of 30 cm for 30 to 240 sec. Subsequently, the fused cells were cultured for 7 to 10 days at 30°C, thereby obtaining bacterial cells (hybrids) formed by cell fusion having stabilized characters.

Hybrids showing a high productivity of vitamin $B_{12}$ of the present invention were selected from the obtained hybrids formed by the cell fusion of two publicly known microorganisms and provided with stabilized characters. The hybrid cell line of the present invention is referred to as Rhodopseudomonas protamicus.

Further, from the selected cells, a specified strain of bacterial cells showed the result of mutual crossing-over of the resistant-character and the susceptible character of the two parent microorganism to antibiotics used as fusion-markers.

The selected group of the hybrid cells formed by the cell fusion of the two microorganism shows the characteristic resistance to colimycin and also to bacitracin, the characteristic susceptibility to cephalothin and the methanol-metabolising property (faint), and such a group of hybrid cells has been identified as a new microorganism by the present inventors as will be described later and has been deposited under the asseccion number of FERM BP-180.

By preliminarily adding each antibiotic and methanol to the culture medium for use in regeneration and selection of the fused protoplasts, it was possible to effectively obtain the strain of hybrid cells having the characters suitable for the object of the present invention.

Concerning the classification of the obtained hybrid, the present inventors carried out the classification according to the descriptions in "Bergey's Manual of Determinative Bacteriology", 8th Ed. and identified the hybrid of the bacterial cells as that belonging to the genus Rhodopseudomonas.

This hybrid of the present invention differs from one of the parent bacteria, Rhodopseudomonas spheroides IFO 12203, in the color tone when cultured respectively in bouillon-agar plate and slant culture and H-II culture medium, in the state of protuberance on the surface and the colour of the colonies, in the utilizability of ammonium nitrate, in the temperature range for growth, in the optimal temperature for growth, in the susceptibility to antibiotics and in the property of metabolizing methanol. Also, this hybrid differs from the other of the parent bacteria, Protaminobacter ruber IFO 3708, in the color tone when cultured respectively in bouillon-agar plate and slant culture and H-II culture medium, in the utilizability of citric acid, in the urease activity thereof, in the temperature range for growth, in the behavior to oxygen, in the reaction to O-F test and in the formation of acids from sugars such as D-xylose and D-fructose.

The thus newly originated hybrid of bacterial cells by cell fusion has been identified as a new microorganism possessing mainly the characters of one of the parent bacteria, Rhodopseudomonas spheroides IFO 12203 and other characters such as the characteristic resistance to two antibiotics, colimycin and bacitracin, the characteristic susceptibility to cephalothin and the methanol-anabolism property which are possessed by the other of the parent bacteria, Protaminobacter ruber IFO 3708 and named as Rhodopseudomonas protamicus by the present inventors.

EXAMPLE 2:

2-1: Production of Cyanocobalamin (CN-vitamin $B_{12}$):

The cell line of Rhodopseudomonas protamicus originated in Example 1, deposited in the Fermentation Research Institute, Agency of Industrial Science and Technology, Japanese Government under the asseccion number of FERM BP-180, was utilized.

The hybrid of the present invention was preliminarily cultured for 5 days in H-II agar culture medium at 30°C.

In a 300 ml-flask for shaking, 100 ml of H-II culture medium were introduced and the culture medium together with the flask were sterilized in autoclave for 20 min at 120°C. Thereafter, one platinum-loop full of the cultured hybrid was inoculated to the prepared H-II culture medium in the flask for shaking and the inoculum was subjected to culture at 30°C for 2 days while placing the flask on a rotary shaker at 180 r.p.m., thereby obtaining a seed culture.

In a 300 ml-flask for shaking, was introduced 100 ml of H-II culture medium and after subjecting the flask to sterilization in autoclave for 20 min at 120°C followed by cooling the flask containing the medium to 30°C, 3 ml of the prepared seed culture were inoculated to the sterilized culture medium in the flask and the inoculum was subjected to shaking culture for 4 days at 30°C while placing the flask on a rotary shaker of 180 r.p.m.

The obtained cultured matter was adjusted to pH 6.0 and after adding potassium cyanide in an amount to make the final concentration of KCN in the mixture to 0.01 % by weight, the mixture was treated in autoclave for 20 min at 120°C under a pressure of 1.2 $kg/cm^2$. After cooling to room temperature, the cooled mixture was subjected to centrifugal separation to obtain a supernatant liquid.

On quantitatively determining the amount of vitamin $B_{12}$ in the cultured matter by the microbiological method while using Lactobacillus leichmannii, it was found that the cultured matter contained 10 mg of vitamin $B_{12}$ per one litre thereof.

Isolation and purification of vitamin $B_{12}$ produced and accumulated in the cultured matter were carried out as follows after transforming vitamin $B_{12}$ to cyanocobalamin (CN-vitamin $B_{12}$) by addition of potassium cyanide thereto.

Two litres of the cultured matter were subjected to centrifugal separation for 20 min at 5°C at 8000 r.p.m. to obtain the bacterial bodies and the supernatant liquid.

To the separated bacterial bodies, was added one litre of 80 % aqueous ethanolic solution (that is, a mixture of 80 % by weight of ethanol and 20 % by weight of water) containing 0.01 % of potassium cyanide and the resultant mixture was stirred at 100°C to extract vitamin $B_{12}$ with the ethanolic solution. The resultant mixture was subjected to centrifugal separation for 20 min at 5°C and at 8000 r.p.m., thereby obtaining a liquid ethanol extract. After condensing the liquid ethanol extract at 40°C under a reduced pressure, and the pH of the residual liquid was adjusted to 4.0. The adjusted residual liquid was subjected to centrifugal separation for 20 min at 5°C and at 8000 r.p.m. to remove the impurities contained in the residual extract.

The supernatant liquid which was an aqueous solution containing vitamin $B_{12}$ at a high concentration was treated as follows.

After passing the supernatant liquid through a column packed with Dowex® 1 X 2 (Cl⁻) (made by Dow Chemical Co.), the liquid passed through the column was adjusted to pH 4.0 and was passed through a column packed with activated carbon to adsorb vitamin $B_{12}$ by the activated carbon.

After washing the column packed with activated carbon with water, vitamin $B_{12}$ adsorbed thereon was eluted by 70 % aqueous acetonic solution (that is, a mixture of 70 % by weight of acetone and 30 % by weight of water). After distilling acetone from the eluted solution, the residual liquid was adjusted to pH 9.0 and was treated by passing through another column packed with Dowex® 1 x 2 (Cl⁻). Then, potassium cyanide was added to the liquid passed through the Dowex column and after adjusting the pH of the resultant mixture to 9.0, the adjusted liquid was further passed through still another Dowex® 1 X 2 (Cl⁻) column to adsorb di-cyanocobalamin. After washing the column with water, the column was treated by a mixture of 0.1N hydrochloric acid and acetone (6:4 by volume) to elute cyanocobalamin adsorbed on the column. After condensing the eluate, acetone was added to the condensate and then, 16 mg of cyanocobalamin was obtained as crystals.

Another method for isolating cyanocobalamin from the cultured matter is set forth below.

Two litres of the cultured matter were subjected to centrifugal separation for 20 min at 5°C at 8000 r.p.m. to obtain the bacterial bodies and the supernatant liquid.

To the separated bacterial bodies, was added one litre of 80 % aqueous ethanolic solution (that is, a mixture of 80 % by weight of ethanol and 20 % by weight of water) containing 0.01 % of potassium cyanide and the resultant mixture was stirred at 100°C to extract vitamin $B_{12}$ with the ethanolic solution. The resultant mixture was subjected to cetrifugal separation for 20 min at 5°C and at 8000 r.p.m., thereby obtaining a liquid ethanol extract. After condensing the liquid ethanol extract at 40°C under a reduced pressure, the pH of the residual liquid was adjusted to 4.0. The adjusted residual liquid was subjected to centrifugal separation for 20 min at 5°C and at 8000 r.p.m. to remove the impurities contained in the residual extract.

- 30 -

The supernatant liquid which was an aqueous solution containing vitamin $B_{12}$ at a high concentration was treated as follows.

After passing the supernatant liquid through a column packed with Dowex® 1 X 2 (Cl⁻) (made by Dow Chemical Co.), the liquid passed through the column was adjusted to pH 4.0 and was passed through the column packed with activated carbon to adsorb vitamin $B_{12}$ by the activated carbon.

After washing the column packed with activated carbon with water, vitamin $B_{12}$ adsorbed thereon was eluted by 70 % aqueous acetonic solution (that is, a mixture of 70 % by weight of acetone and 30 % by weight of water). After distilling acetone from the eluted solution, the residual liquid was subjected to extraction with 50 ml of a mixture of p-chlorophenol and chloroform (2:3 by volume). After washing the extract with 2 times by volume of water, 100 ml of a mixture of p-chlorophenol, n-butanol and water (1:1:1 by volume) were added to the washed extract. The formed mixture was shaken to transfer vitamin $B_{12}$ from the organic layer to the aqueous layer. After washing the aqueous layer with chloroform and then with diisopropyl ether, the aqueous layer was condensed and thereafter, acetone was added to the condensate and then, 16 mg of cyanocobalamin was obtained as crystals.

2-2:  Production of Methylcobalamin ($CH_3-B_{12}$):

Isolation and purification of vitamin $B_{12}$ in the form

as it has been formed in the cultured matter were carried out in the dark without adding cyanide ions to the cultured matter as follows:

After subjecting 2 litres of a cultured matter obtained by the same procedures as above to centrifugal separation into the bacterial bodies and the supernatant liquid, one litre of 80 % aqueous ethanolic solution (that is, a mixture of 80 % by weight of ethanol and 20 % by weight of water) was added to the separated bacterial bodies, and the mixture was stirred for 20 min at 100°C to extract vitamin $B_{12}$ in the bacterial bodies with ethanol.

After adding 200 ml of a mixture of p-chlorophenol and chloroform (2:3 by volume) to the ethanolic extract liquid, the obtained mixture was shaken. The mixture was left to stand still, the underlaying p-chlorophenol layer was washed with a small amount of water and after adding the same volume of a mixture of chloroform, n-butanol and water (1:1:1) to the p-chlorophenol layer, the mixture was shaken and then was left to stand still.

Vitamin $B_{12}$ was transferred from the p-chlorophenol layer to the aqueous layer, thereby obtaining a concentrated aqueous solution of vitamin $B_{12}$.

After washing the aqueous solution with a small amount of chloroform and then with isopropyl ether, the pH of the aqueous solution was adjusted to pH of 3.5, and the aqueous solution was passed through a column packed with Amberlite®
IRC-50 (made by Rohm & Haas Co., USA) and the column was eluted
with at first a small amount of water and then with an aqueous
ammonia solution of pH 9.5 to obtain a fraction containing
DBCC and methylcobalamin (also called as $CH_3-B_{12}$).

After purifying the obtained fraction by the same
procedures as above of using p-chlorophenol, DBCC and $CH_3-B_{12}$
were separated by column-chromatography while using CM-cellulose.
By adding acetone to the respective eluates, 10 mg of DBCC and
6 mg of $CH_3-B_{12}$ were obtained both as crystals.

EXAMPLE 3:

In a 30 litre-jar type fermentor, were introduced
20 litres of a culture medium which was prepared by dissolving,
in each one litre of de-ionized water, 30 g of glucose, 10 g
of polypeptone, 10 g of yeast extract, 1.5 g of urea, 1.5 g
of ammonium sulfate, 1.5 g of potassium dihydrogen phosphate,
1.5 g of dipotassium hydrogen phosphate, 0.3 g of magnesium
sulfate heptahydrate, 0.1 g of calcium chloride dihydrate,
0.1 g of zinc sulfate heptahydrate, 0.1 g of ferrous sulfate
heptahydrate, 0.01 g of manganese sulfate heptahydrate, 0.1 g
of cobalt chloride hexahydrate and 0.2 ml of ADEKANOL® LG-126
(made by Asahi Denka Kogyo Co.) as an antifoaming agent. The
culture medium was sterilized for 30 min at 120°C under a pressure
of 1.2 $kg/cm^2$G.

After cooling the culture medium to room temperature,
a seed culture prepared in the same manner as in Example 1 was inoculated to the sterilized culture medium at a volume rate of 2 %, and the microorganism, of the present invention was cultured at 30°C for 72 hours while stirring the culture medium at 350 r.p.m., while carring out an aeration at a rate of 10 litres/min and automatically regulating the pH of the culture medium in a range from 7.0 to 7.5 by 14 % aqueous ammonia solution. It was found that the formation of vitamin $B_{12}$ during 72 hours of aerobic culture was 44 mg/litre of the culture medium.

The cultured matter was subjected to centrifugal separation for 20 min at 8000 r.p.m. at 5°C into the bacterial bodies and the supernatant liquid. Thereafter, the bacterial bodies were suspended in 10 litres of 80 % aqueous ethanolic solution containing 0.01 % by weight of potassium cyanide and vitamin $B_{12}$ was extracted while stirring the ethanolic solution for 20 min at 100°C.

After cooling the mixture, it was subjected to centrifugal separation and the extraction residue was subjected to extraction in the same manner as above. After combining the two liquid extracts, the combined liquid extract was condensed under a reduced pressure to evaporate ethanol from the extract. After adjusting the pH of the condensate to 4.0, the condensate was subjected to centrifugation to remove the precipitated matter.

Thereafter, the obtained supernatant liquid was treated with 400 g of activated carbon and after washing the activated carbon with water, the absorbed matter on the activated carbon was eluted with 75% aqueous acetonic solution (that is, a mixture of 75% by weight of acetone and 25% by weight of water).

After condensing the eluate under a reduced pressure and adjusting the pH of the condensate to 4.0, the condensate was passed through a column packed with 200 g of an (strongly basic)anion-exchange resin, Dowex® 1 X 2 ($Cl^-$), which had been adjusted to be Cl-type.

The passed liquid was passed through the column packed with 200 g of Amberlite® IRC-50 which had been adjusted to pH of 3.5 with aqueous 0.1N hydrochloric acid solution to adsorb vitamin $B_{12}$. After washing the column with a small amount of water, the substance adsorbed on the column was eluted by aqueous ammonia solution of pH 9.0 to collect the fraction containing vitamin $B_{12}$.

After neutralizing the collected fraction containing vitamin $B_{12}$ with aqueous 0.2N hydrochloric acid solution, the neutralized fraction was passed through a new column packed with activated carbon to adsorb vitamin $B_{12}$. After washing the activated carbon column with water, the column was treated with 75 % aqueous acetonic solution to elute vitamin $B_{12}$. By condensing the eluate under a reduced pressure, acetone was distilled off from the eluate so as to obtain an aqueous solution containing vitamin $B_{12}$ at a high concentration.

Thereafter, acetone was added to the condensate and 650 mg of vitamin $B_{12}$ as crystals was obtained.

On the other hand, the supernatant liquid which had been separated from the cultured matter was condensed to dryness, and vitamin $B_{12}$ was extracted therefrom by the same procedures of extracting vitamin $B_{12}$ from the bacterial bodies. After purification thereof, 160 mg of vitamin $B_{12}$ were obtained as crystals.

In short, from 20 litres of the cultured matter of Rhodopseudomonas protamicus, 810 mg of vitamin $B_{12}$ (cyanocobalamin, referred to as $CN-B_{12}$) was obtained (40.5 mg of $CN-B_{12}$/one litre of the cultured matter).

The purity of the thus obtained vitamin $B_{12}$ was higher than 99.0 % according to the determination by liquid-chromatographic method, spectrocalorimetric method and the microbiological method while using Lactobacillus leichmannii.

EXAMPLE 4:

In a 30 litre-jar type fermentor, were introduced 20 litres of a culture medium which was prepared by dissolving, in each one litre of de-ionized water, 50 g of glucose, 20 g of polypeptone, 30 g of yeast extract, 3 g of urea, 3 g of ammonium sulfate, 3 g of potassium dihydrogen phosphate, 3 g of dipotassium hydrogen phosphate, 0.6 g of magnesium sulfate heptahydrate, 0.2 g of calcium chloride dihydrate, 0.2 g of zinc sulfate heptahydrate, 0.2 g of ferrous sulfate heptahydrate, 0.02 g of manganese sulfate heptahydrate, 0.2 g of cobalt chloride hexahydrate and 0.4 ml of ADEKANOL® as an antifoaming agent. After sterilizing the culture medium in a same manner as in Example 3 and cooling the sterilized culture medium to room temperature, a seed culture prepared in the same manner as in Example 1 was inoculated to the culture medium and was cultured in the same manner as in Example 3 except for the duration of culture of 90 hours in Example 3.

By extracting and purifying the cultured matter in the same manner as in Example 3, 2.7 g of vitamin $B_{12}$ (cyano-cobalamin, referred to CN-$B_{12}$) were obtained. The yield corresponds to 135 mg of CN-$B_{12}$/one litre of the cultured matter and 2.7 mg of CN-$B_{12}$/one gram of dried bacterial bodies.

EXAMPLE 5:

After preparing a culture medium in the same manner as in Example 2, a preliminarily cultured <u>Rhodopseudomonas protamicus</u> was inoculated to the prepared culture medium as a seed culture and in the same time, the sterilized and cooled liquid paraffin was placed on the liquid surface of the culture medium so as to make a layer of 1 cm in thickness. Thereafter, a stationary and anaerobic culture was carried out at 30°C for 7 days while exposing the culture medium to a light from a luminescent lamp at a height of 30 cm above from the surface of the culture medium.

After ending culturing, liquid paraffin was removed, and the cultured matter was treated in the same manner as in Example 2 to obtain vitamin $B_{12}$ at a rate of 8.2 mg/one litre of the cultured matter.

<u>COMPARATIVE EXAMPLE</u>:

Three bacterial, <u>Protaminobacter</u> <u>ruber</u>  IFO 3708 (one of the parent bacteria of <u>Rhodopseudomonas</u> <u>protamicus</u> FERM BP-180 of the present invention), <u>Rhodopseudomonas</u> <u>spheroides</u>  IFO 12203 (the other of the parent bacteria of <u>Rhodopseudomonas</u> <u>protamicus</u> FERM BP-180 of the present invention) and <u>Propionibacterium</u> <u>freudenreichii</u> which is used practically for producing vitamin $B_{12}$, were respectively cultured in the same manner as in Example 2 for 72 hours to obtain vitamin $B_{12}$ in the respective amounts shown in the following table:

Table: Amounts of Vitamin $B_{12}$ produced respectively
by the three Bacteria

| Bacteria | | Amounts of dried bacterial bodies (g/ℓ*) | Amounts vitamin $B_{12}$ (μg/ℓ*) | Content of vitamin $B_{12}$ in bacterial bodies (μg/g**) |
|---|---|---|---|---|
| The present invention | Rhodopseudomonas protamicus FERM BP-180 | 6.5 | 10,000 | 1,538 |
| Comparative example | Protaminobacter ruber IFO 3708 | 5.5 | 358 | 65 |
| | Rhodopseudomonas spheroides IFO 12203 | 5.0 | 100 | 20 |
| | Propionibacterium freudenreichii | 5.5 | 1,375 | 250 |

Notes:   *ℓ of the cultured matter

**g of dried bacterial bodies

## CLAIMS

1. A method for producing a fused cell hybrid, which method comprises fusing a cell of a chlorophyll-producing bacterial species belonging to the photosynthetic genus Rhodopseudomonas with a cell of a vitamin $B_{12}$-producing bacterial species belonging to the methanol-metabolising genus Protaminobacter.

2. A method according to claim 1, in which a cell of a microorganism belonging to the species Rhodopseudomonas spheroides is fused with a cell of a microorganism belonging to the species Protaminobacter ruber.

3. A method according to claim 1 or 2, in which the fused cell which is obtained is exposed to ultraviolet rays.

4. A fused cell hybrid which has been produced by a process as claimed in any one of the preceding claims and which produces vitamin $B_{12}$.

5. The fused cell hybrid Rhodopseudomonas protamicus (FERM BP-180).

6. A process for producing vitamin $B_{12}$, which process comprises culturing a fused cell hybrid as claimed in claim 4 or 5 and collecting the vitamin $B_{12}$ thus-produced.

7. A pharmaceutical composition comprising as active ingredient vitamin $B_{12}$ which has been produced by a process as claimed in claim 6 together with a pharmaceutically acceptable carrier or diluent.

Figure

0131456

1/1